# EUROPÄISCHE PATENTSCHRIFT

(11) **EP 3 785 633 B1**
(45) Veröffentlichungstag und Bekanntmachung des Hinweises auf die Patenterteilung: **18.10.2023**
(21) Anmeldenummer: 20193743.0
(22) Anmeldetag: 31.08.2020
(51) Int. Cl.: A61B 5/145, A61B 5/1459, A61B 5/1473, G01N 31/22, G01N 21/78, G06T 7/90, G01N 33/03

(54) **VERFAHREN UND VORRICHTUNG ZUM AUSWERTEN EINES FARBINDIKATORS**
METHOD AND APPARATUS FOR EVALUATING A COLOUR INDICATOR
PROCÉDÉ ET DISPOSITIF D'ÉVALUATION D'UN INDICATEUR CHROMATIQUE

(30) Priorität: 02.09.2019 DE 102019123474
(43) Veröffentlichungstag der Anmeldung: 03.03.2021
(73) Patentinhaber: Testo SE & Co. KGaA, 79853 Lenzkirch (DE)
(72) Erfinder: Landenberger, Benjamin, 79102 Freiburg (DE); Kneer, Janosch, 79115 Freiburg (DE); Zubler, Martin, 79853 Lenzkirch (DE)
(74) Vertreter: Mertzlufft-Paufler, Cornelius

(56) Entgegenhaltungen:
- WO-A1-2013/116831
- WO-A1-2016/102337
- US-A1- 2006 092 030
- US-A1- 2013 303 869
- US-A1- 2017 184 506

## Beschreibung

Die Erfindung betrifft ein Verfahren zum Bestimmen des Messwertes einer Messgröße einer Substanz mit Hilfe eines Farbindikators, wobei der Farbindikator wenigstens ein Indikatorfeld aufweist, das zuvor mit der Substanz in Kontakt gebracht wurde.

Ein solches Verfahren kann beispielsweise zur Untersuchung einer Flüssigkeit angewandt werden, wobei auf einem Teststreifen oder Testträger eine Substanz in einem Indikatorfeld vorgehalten ist, die in Abhängigkeit von einer Eigenschaft der Flüssigkeit eine Farbänderung oder ein Farbumschlag zeigt.

Derartige Untersuchungsverfahren sind bekannt und werden beispielsweise zur Untersuchung des Anteils von freien Fettsäuren oder von polaren Anteilen in Frittieröl verwendet. Farbumschläge dieser Art sind jedoch auch gebräuchlich bei der Untersuchung von anderen Substanzen und Messwerten, beispielsweise den pH-Wert einer Flüssigkeit. Es hat sich hierbei bewährt, dem Benutzer eine Farbskala als Farbreferenz mitzugeben, mit welcher er eine Farbänderung oder Färbung des Indikatorfeldes bewerten kann.

Dies hat allerdings den Nachteil, dass derartige Farbänderungen graduell erfolgen, sodass eine genaue Einordnung, um wie viel die Farbänderung erfolgt ist, schwierig ist. Zudem stimmen die Farben der Indikatorfelder nicht exakt mit der Farbskala überein, wodurch eine Zuordnung zu einem Messwert zusätzlich erschwert wird.

Die Erfindung hat demnach die Aufgabe eine einfachere und insbesondere wesentlich genauere Bestimmung eines Messwertes zu ermöglichen.

Diese Aufgabe wird erfindungsgemäß durch die Verfahrensschritte gemäß dem Anspruch 1 gelöst.

Das erfindungsgemäße Verfahren ist demnach dadurch gekennzeichnet, dass der Farbindikator und eine Farbreferenz bildlich erfasst werden, wobei die Farbreferenz wenigstens zwei verschiedenfarbige Referenzfelder aufweist, dass computerunterstützt anhand der aufgenommenen Farbwerte der Referenzfelder und des Indikatorfeldes ein Zwischenwert der Messgröße ermittelt wird und dass der Messwert der Substanz aus diesem Zwischenwert ermittelt wird.

Das bildliche Erfassen kann beispielsweise mit einer herkömmlichen Digitalkamera oder einem Smartphone erfolgen.

Der Vorteil besteht nun darin, dass die Farbwerte der Farbindikatorfelder nicht mehr visuell erfasst und mit einer Farbreferenz verglichen werden müssen.

Vielmehr wird aus dem Bild der Farbwert des Indikatorfeldes ermittelt. Anhand der aufgenommenen Farbwerte der Referenzfelder wird computerunterstützt dieser Farbwert des Indikatorfeldes einem Zwischenwert zugewiesen. Der Zwischenwert besitzt vorzugsweise dieselbe physikalische Dimension wie der zu bestimmende Messwert. Aus dem Zwischenwert wird dann computerunterstützt der Messwert ermittelt.

Insbesondere vorteilhaft ist es, wenn die Indikatorfelder und die Referenzfelder automatisch erkannt werden, beispielsweise durch eine Muster- oder Kantenerkennung oder anhand einer vorgegebenen Lage in Bezug auf eine Lagereferenz. Eine solche Lagereferenz kann beispielsweise gesondert gekennzeichnet sein, so dass sie eindeutig identifiziert werden kann.

Je nach Farbindikator nehmen die Indikatorfelder unterschiedliche Farben an. Die Farben können dabei graduell variieren oder diskrete Farbwerte annehmen.

Erfindungsgemäß definieren jeweils zwei verschiedenfarbige Referenzfelder die Endfarben einer Farbskala. Auf diese Weise ist durch wenige Referenzfelder eine ganze Skala der potentiellen Farben der Indikatorfelder definierbar. Erfindungsgemäß sind die Endfarben jeweils einem Endwert des Messbereichs des Farbindikators zugeordnet. Bei einem pH-Farbindikator kann der Messbereich beispielsweise zwischen einem pH-Wert von 1 bis 10 liegen. Dementsprechend können die Endfarben etwa blau für pH 10 und rot für pH 1 sein. Zwischenwerte des Messbereichs können auf diese Weise der Farbskala entnommen oder zugeordnet werden.

Erfindungsgemäß wird der Zwischenwert durch Interpolation des Farbwertes des Indikatorfeldes innerhalb der Farbskala und der zugeordneten Endwerte ermittelt

Die Farbindikatoren unterliegen vielfältigen Schwankungen, beispielsweise durch Produktion und/oder Anwendung, so dass die ermittelten Zwischenwerte einer großen statistischen Schwankung unterliegen. In einer besonders vorteilhaften Ausführung erfolgt das Ermitteln des Zwischenwertes und/oder des Messwertes aus dem Zwischenwert anhand von statistischen Methoden und/oder durch eine künstliche Intelligenz. Auf diese Weise kann durch Mittelung über viele Einzelmessungen eine statistische Anpassung erfolgen, so dass ein genauerer Messwert ermittelt wird. Insbesondere bei der Verwendung einer künstlichen Intelligenz (KI) ist eine signifikante Verbesserung der Messgenauigkeit möglich.

Die künstliche Intelligenz kann als Eingang den Farbwert und/oder Zwischenwert jedes Indikatorfeldes und den Farbwert jedes Referenzfeldes erhalten und als Ausgang den Messwert der Substanz liefern. Auf diese Weise können sehr viele Umgebungseinflüsse in die Ermittlung des Messwertes einfließen. Die künstliche Intelligenz ist aber demnach jeweils immer nur für genau eine Kombination aus Farbindikator und Farbreferenz einsetzbar. Das bedeutet, dass für einen Farbindikator, der eine andere Messgröße und andere Farbwerte aufweist eine eigene künstliche Intelligenz trainiert werden muss.

In einer vorteilhaften Weiterbildung kann jeder ermittelte Messwert zum weiteren Training der künstlichen Intelligenz verwendet werden.

Zur Verbesserung der Bestimmung der Farbwerte kann es vorteilhaft sein, wenn ein automatischer Farbabgleich, insbesondere Weißabgleich, vorgenommen wird. Der Farbabgleich kann beispielsweise durch eine interne Kameralogik bei oder nach der Bildaufnahme vorgenommen werden.

In einer vorteilhaften Ausführung weist die Farbreferenz wenigstens ein Farbabgleichsfeld auf, das als Basis für den Farbabgleich dient. Dieses Farbabgleichsfeld kann beispielsweise weiß für einen Weißabgleich oder grau für einen Grauabgleich sein. Der Farbabgleich kann beispielsweise erfolgen, bevor die Farbwerte der Indikatorfelder und der Referenzfelder bestimmt werden.

Es ist jedoch auch möglich, dass die Farbwerte der Farbabgleichsfelder als weitere Eingänge der künstlichen Intelligenz dienen. Auf diese Weise erfolgt der automatische Farbabgleich durch die künstliche Intelligenz.

Prinzipiell ist es möglich, den Farbindikator und die Farbreferenz in separaten Bildern zu erfassen. Jedoch haben Aufnahmeparameter wie Kamerapose, Abstand zum Objekt, Winkel zum Objekt, Brennweite, Blende, Belichtungszeit, ISO-Empfindlichkeit, Beleuchtung usw. Einfluss auf die Ermittlung der Farbwerte. Um diese Aufnahmeparameter gleich zu halten, ist es daher zweckmäßig, wenn die bildliche Erfassung des Farbindikators und der Farbreferenz gleichzeitig, insbesondere in einem Bild, erfolgt. Dadurch ist eine Variation der oben genannten Aufnahmeparameter minimiert oder ausgeschlossen, so dass die Ermittlung der Farbwerte genauer erfolgen kann.

Für eine erhöhte Genauigkeit bei der Auswertung ist es vorteilhaft, wenn der Farbindikator mehr als ein Indikatorfeld aufweist. Auf diese Weise können beispielsweise fertigungstechnische Abweichungen der Indikatorfelder in die Ermittlung des Messwertes mit einfließen.

Zweckmäßig kann es daher sein, wenn der Farbindikator wenigstens zwei, insbesondere vier, Indikatorfelder aufweist. Diese Indikatorfelder können gleich sein, so dass für die Auswertung jeweils mehrere redundante Informationen zur Verfügung stehen und damit ein genaueres Ergebnis erzielbar ist.

In einer Ausführung der Erfindung besitzen die Indikatorfelder unterschiedliche Sensitivitäten gegenüber der zu messenden Substanz. Demnach wechseln die einzelnen Indikatorfelder bei verschiedenen Messwerten ihre Farbe, so dass der Farbindikator eine räumliche Dimension erhält, wodurch eine zusätzliche Verbesserung der Genauigkeit erreicht werden kann.

In einer vorteilhaften Ausführung weist die Farbreferenz mehr als ein Referenzfeld für jede Endfarbe auf. Diese können beispielsweise entsprechend zu den Indikatorfeldern auf dem Farbindikator voneinander beabstandet sein. Auf diese Weise können Ausleuchtungsunterschiede der Indikatorfelder innerhalb des Bildes und beispielsweise geometrische Verzerrungen berücksichtigt und kompensiert werden.

Besonders zweckmäßig ist es, wenn die mehreren Referenzfelder einer Endfarbe jeweils identische Farben aufweisen. Insbesondere zweckmäßig ist es, wenn für jede Endfarbe zwei Referenzfelder vorhanden sind, die voneinander beabstandet und/oder abwechselnd angeordnet sind. Durch eine abwechselnde Anordnung kann eine bessere Verteilung der Referenzfelder erzielt werden, so dass Abweichungen im Bild wie oben beschrieben kompensierbar sind.

Ein Farbindikator ist in der Regel dazu ausgebildet in eine Flüssigkeit getaucht zu werden oder mit einer Flüssigkeit benetzt zu werden. Dadurch können je nach Aufnahmepose Reflexionen entstehen, die im schlechtesten Fall ein Auswerten der Farbe eines Indikatorfelds oder eines Referenzfeldes erschweren oder unmöglich machen.

In einer Ausführung ist der Farbindikator deshalb so ausgebildet, dass die zu messende Substanz so gebunden wird, dass keine Reflexionen durch Umgebungslicht entstehen. Dazu kann ein Grundträger des Farbindikators saugfähig sein oder eine raue Oberfläche aufweisen.

In einer Ausführung können Mittel zum Erkennen störender Reflexionen vorhanden sein, so dass beispielsweise eine Warnung ausgegeben werden kann. Solche Mittel können beispielsweise bereits vor der Bildaufnahme Reflexionen erkennen und den Benutzer darauf hinweisen, so dass die Kamerapose und/oder die Beleuchtung gewechselt werden kann.

Es kann auch vorgesehen sein, dass Reflexionen aus einem bestehenden Bild durch entsprechende Bildbearbeitungsalgorithmen herausgerechnet werden, wodurch jedoch die Genauigkeit leiden kann.

Prinzipiell ist es möglich, dass der Farbindikator und die Farbreferenz einteilig oder mehrteilig ausgebildet sind. Vorteilhaft ist es dabei, wenn die Farbreferenz separat ausgebildet ist, da diese dann mehrfach wiederverwendbar ist. In einer zweckmäßigen Ausführung sind die Größe und die Abstände der Referenzfelder der Größe beziehungsweise den Abständen der Indikatorfelder angepasst, insbesondere wobei die Abstände identisch sind, so dass die Referenzfelder jeweils neben ein Indikatorfeld platzierbar sind. Dadurch kann die Genauigkeit der erfassten Farbwerte verbessert werden, da die Farbwerte, aufgrund der lokal gleichen Beleuchtung, eine geringere Abweichung aufweisen können.

Die Erfindung umfasst weiter eine Vorrichtung zum Bestimmen des Messwertes einer Eigenschaft einer Substanz mit Hilfe eines Farbindikators. Diese Vorrichtung ist erfindungsgemäß dadurch gekennzeichnet, dass die Vorrichtung eine Bilderfassungseinheit zur bildlichen Erfassung des Farbindikators und einer Farbreferenz, und eine Auswerteeinheit zur computerunterstützten Ermittlung eines Zwischenwertes anhand der aufgenommenen Farbwerte der Referenzfelder der Farbreferenz und eines Indikatorfeldes des Farbindikators und zur Ermittlung des Messwerts der Substanz aus diesem Zwischenwert aufweist.

Die erfindungsgemäße Vorrichtung ist in Anspruch 10 definiert.

Besonders vorteilhaft ist die Vorrichtung zur Durchführung eines erfindungsgemäßen Verfahrens ausgebildet.

Die Vorrichtung kann als speziell angepasstes Messgerät ausgebildet sein. Besonders vorteilhaft ist es jedoch, wenn die Vorrichtung ein Smartphone, ein Tablet oder ein Notebook ist, bei dem die Auswerteeinheit durch ein Softwareprogramm oder eine App ausgebildet ist. Smartphones sind weit verbreitet und besitzen in der Regel eine Bildaufnahmeeinheit, so dass eine erfindungsgemäße Ermittlung eines Messwertes einfach und kostengünstig durchführbar ist. Der weitere Vorteil besteht darin, dass Updates und/oder Verbesserungen an der Messwertermittlung jederzeit durch ein Softwareupdate umsetzbar sind.

Die Erfindung ist nachfolgend anhand einer beispielhaften Ausführung mit Bezug auf die beiliegenden Zeichnungen näher erläutert.

Es zeigt:
- Fig. 1:: einen Farbindikator und eine Farbreferenz in Form von Teststreifen,
- Fig. 2:: ein Ablaufdiagramm eines erfindungsgemäßen Verfahrens und

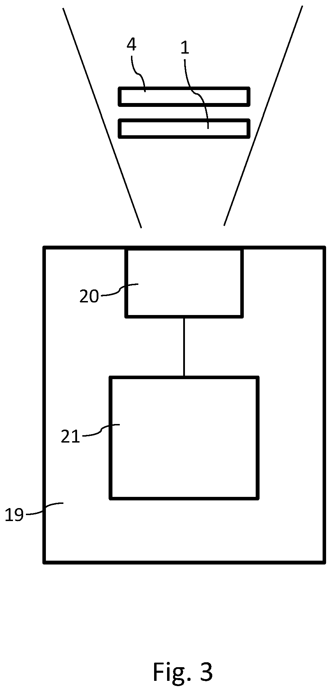

- Fig. 3:: eine erfindungsgemäße Vorrichtung.

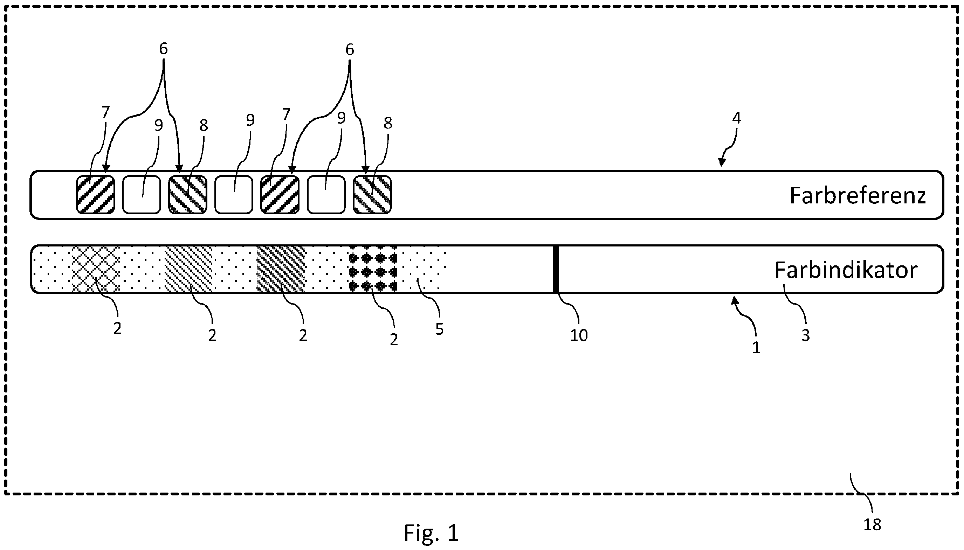

Die Figur 1 zeigt einen Farbindikator 1 zum Eintauchen in eine Flüssigkeit. Ein solcher Farbindikator 1 kann beispielsweise zur Bestimmung des pH-Wertes oder anderer Messgrößen eingesetzt werden.

Im Beispiel ist ein Farbindikator 1 zur Bestimmung der freien Fettsäuren in Frittieröl gezeigt. Ein solcher Farbindikator ist beispielsweise als Teststreifen von 3M^{™} unter der Bezeichnung LRSM 200 verfügbar. Der Farbindikator 1 besitzt vier Indikatorfelder 2, die äquidistant voneinander beabstandet auf einem Grundträger 3 angeordnet sind.

Die Indikatorfelder 2 besitzen bei diesem Farbindikator 1 unterschiedliche Sensitivitäten, so dass ein bestimmter Messwert durch die Anzahl der Indikatorfelder 2 mit Farbumschlag bestimmt werden kann. Dabei erfolgt der Farbumschlag jedoch zum Teil graduell.

Der Farbindikator 1 besitzt eine Markierung 10, die eine maximale Eintauchtiefe anzeigt.

Die Fig. 1 zeigt weiter eine Farbreferenz 4, die ebenfalls auf einem streifenförmigen Grundträger 3 angeordnet ist. Die Farbreferenz 4 weist im Beispiel vier Referenzfelder 6 auf. Zwei der Referenzfelder 6 besitzen eine erste Endfarbe 7 und die beiden anderen Referenzfelder 6 besitzen eine zweite Endfarbe 8. Die Referenzfelder 6 mit den beiden Endfarben sind dabei abwechselnd auf dem Grundträger 3 angeordnet.

Im Beispiel ist jeweils zwischen zwei benachbarten Referenzfeldern 6 ein weißes Farbabgleichsfeld 9 angeordnet, das für einen automatischen Weißabgleich eines Bildes verwendet werden kann. Die Farbabgleichsfelder 9 besitzen im Beispiel die gleichen Abmessungen wie die Referenzfelder 6.

Insbesondere besitzen die Referenzfelder 6 die gleichen Abstände zueinander wie die Indikatorfelder 2, so dass jeweils ein Referenzfeld 6 neben ein Indikatorfeld 2 anordenbar ist, wie in Fig. 1 gezeigt. Dadurch können Farbabweichungen aufgrund von ungleichmäßiger Beleuchtung reduziert und damit die Farbzuordnung verbessert werden. Die Farbreferenz kann jedoch auch an anderer Stelle platziert werden.

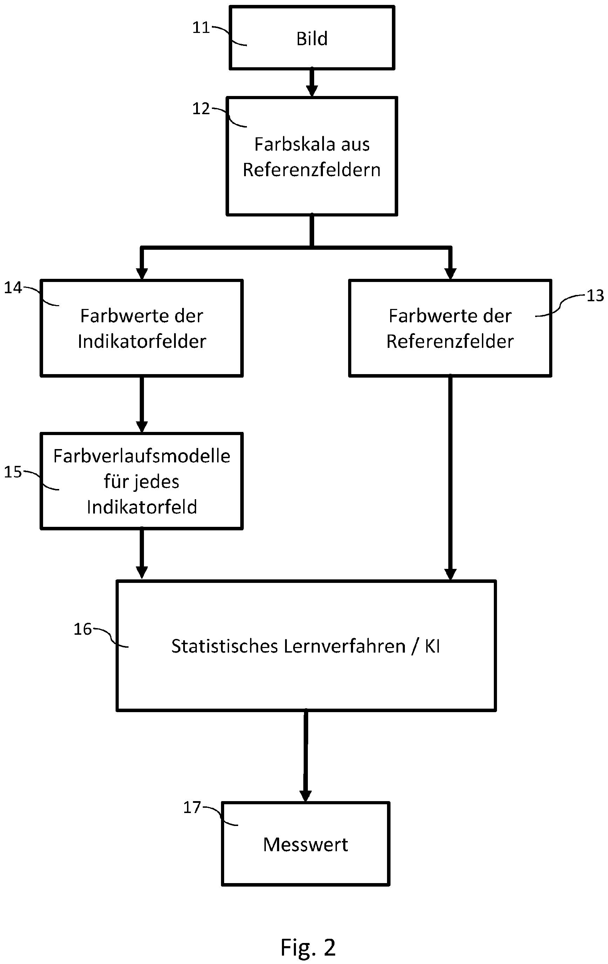

Die Fig. 2 zeigt ein Ablaufdiagramm eines vorteilhaften Verfahrens gemäß der Erfindung. Im Beispiel soll der Gehalt an freien Fettsäuren in einem Frittierfett oder Frittieröl einer Fritteuse bestimmt werden. Dazu werden die oben beschriebenen LRSM 200 Teststreifen als Farbindikator verwendet. Das Verfahren ist ohne Änderung auch zur Bestimmung von anderen Messgrößen unter Verwendung von anderen Farbindikatoren anwendbar.

Der Farbindikator 1 wird dazu zunächst bis zur Markierung 10 in die zu testende Flüssigkeit, im Beispiel das Frittierfett, eingetaucht, so dass alle Indikatorfelder 2 mit Frittierfett 5 benetzt sind.

In einem Bilderfassungsschritt 11 werden der Farbindikator 1 und eine Farbreferenz 4 bildlich erfasst. Vorzugsweise werden der Farbindikator 1 und die Farbreferenz 4 gleichzeitig in einem Bild 18 erfasst.

Dazu können der Farbindikator 1 und die Farbreferenz 4 nebeneinander auf eine Oberfläche platziert werden, wie beispielsweise in Fig. 1 gezeigt. Dies hat den Vorteil, dass alle Aufnahmeparameter, wie etwa die Kameraperspektive und die Beleuchtung, für Farbindikator 1 und Farbreferenz 4 gleich sind, so dass eventuell störende Einflüsse eine geringere Relevanz aufweisen oder herausgerechnet werden können.

Vorzugsweise ist die Farbreferenz 4 dem Farbindikator 1 so angepasst, dass die Referenzfelder 6 etwa die gleichen Abmessungen und die gleichen Abstände aufweisen, wie die Indikatorfelder 2 des Farbindikators 1.

Es ist jedoch auch möglich den Farbindikator 1 und die Farbreferenz 4 separat aufzunehmen, wodurch jedoch die oben genannten Vorteile eventuell nicht vorhanden sind.

Besonders zweckmäßig ist es dabei, wenn das Bild digital erfasst wird, beispielsweise mit einer Smartphone-Kamera oder zumindest in digitaler Form dem Verfahren bereitgestellt wird.

Das erfindungsgemäße Verfahren kann in einem separaten Gerät oder als Anwendungsprogramm zur Ausführung auf einem mobilen Gerät, insbesondere einem Smartphone oder einem Tablet-Computer, ausgebildet sein.

In einem Farbskalaschritt 12 wird aus den Referenzfeldern 6 eine Farbskala ermittelt. Die Farbskala wird dabei durch die erste Endfarbe 7 und die zweite Endfarbe 8 definiert.

Zwischenfarbwerte der Farbskala werden dabei durch Farbinterpolation bestimmt. Hierbei können verschiedene Farbmodelle verwendet werden, wobei die Wahl des Farbmodells von der Art der Farbindikatoren abhängen kann.

Je nach Art des Farbindikators kann die Farbskala auch durch mehr als zwei Endfarben definiert sein, insbesondere wobei eine oder mehrere Zwischenfarben definiert sind. Die Anzahl der notwendigen Referenzfelder erhöht sich dadurch eventuell.

In einem Farbabgleichsschritt 13 werden die Farbwerte der Farbabgleichsfelder 9 ermittelt. Die Farbwerte können dabei in einem üblichen Format, etwa als RGB- oder HSV-Werte, aus dem digitalen Bild 18 ausgelesen werden.

Diese Farbabgleichsfelder 9 weisen vorzugsweise jeweils die gleiche Farbe auf und dienen zum Farbabgleich des aufgenommenen Bildes 18. Die Farbabgleichsfelder 9 können beispielsweise weiß für einen automatischen Weißabgleich oder grau für einen Grauabgleich sein. Es können jedoch auch weiße und graue Farbabgleichsfelder 9 vorhanden sein, so dass ein doppelter Farbabgleich durchgeführt werden kann. Es kann auch ein farbiges Farbabgleichsfeld vorhanden sein, beispielsweise in einer zu den beiden Endfarben komplementären Farbe, wodurch ebenfalls ein genauer Farbabgleich möglich ist. Durch die beabstandete Anordnung von mehreren Farbabgleichsfeldern 9 ist zusätzlich ein Ausgleich einer ungleichmäßigen Ausleuchtung des Bildes möglich, die beispielsweise bei einer schrägen Bildaufnahme durch unterschiedliche Abstände entstehen kann.

In einem Indikatorschritt 14 werden die Farbwerte der Indikatorfelder 2 ermittelt. Die Farbwerte werden hier zweckmäßigerweise im selben Format ermittelt, wie die Farbwerte der Farbabgleichsfelder 9.

Zur automatischen Ermittlung der Farbwerte der Indikatorfelder 2, der Referenzfelder 6 und der Farbabgleichsfelder 9 kann ein Verfahren zur Mustererkennung, Kanten- und/oder Eckenerkennung oder ein anderes bekanntes Verfahren zur Objekterkennung angewendet werden. Der Vorteil dabei ist, dass der Farbindikator 1 und die Farbreferenz 4 in nahezu beliebiger Lage und Anordnung zueinander bildlich erfasst werden können. Dadurch wird die Anwendung sehr einfach und wenig fehleranfällig.

Es kann jedoch auch auf dem Farbindikator 1 und/oder der Farbreferenz 4 eine Lagereferenz vorhanden sein, von der die relative Lage zu den anderen Feldern bekannt ist. Eine solche Lagereferenz kann beispielsweise ein spezielles Symbol oder etwa ein QR-Code sein.

Es ist auch möglich, dass die Farbreferenz 4 in einer definierten Lage zum Farbindikator 1 angeordnet ist. Die Farbreferenz 4 kann beispielsweise eine Aussparung zum Einlegen des Farbindikators 1 aufweisen.

In einem Modellschritt 15 wird für jedes Indikatorfeld 2 anhand des Farbwertes und eines dem Indikatorfeld 2 zugeordneten Farbverlaufsmodell ein Zwischenwert bestimmt. Der Zwischenwert kann dabei bereits die physikalische Dimension des zu bestimmenden Messwertes besitzen.

Diese Zwischenwerte sind einer großen statistischen Schwankung unterlegen. Insbesondere da die Sensitivitäten der einzelnen Indikatorfelder nicht genau herstellbar sind und weil die Farbwechsel nur graduell erfolgen.

Daher ist eine analytische Bestimmung eines Messwertes nur schwer möglich. In einem Auswerteschritt 16 wird daher auf Grundlage einer künstlichen Intelligenz (KI) aus den Ausgangsdaten ein Messwert ermittelt.

Die KI basiert auf einem statistischen, maschinellen Lernverfahren, das durch viele Einzelmessungen trainiert wurde. Insbesondere besitzt die KI als Eingangsparameter alle ermittelten Farbwerte und Zwischenwerte. Demnach ist eine solche KI nur zur Auswertung eines bestimmten Farbindikators zusammen mit einer Farbreferenz ausgebildet.

Andere Farbindikatoren mit einer unterschiedlichen Anzahl an Indikatorfeldern benötigen jeweils eine eigens trainierte KI.

Es kann zweckmäßig sein, wenn jeder durchgeführte Messvorgang zum weiteren Training der KI verwendet wird. Dazu kann es vorteilhaft sein, wenn die KI beispielsweise auf einem zentralen Server als Webdienst ausgebildet ist, so dass alle Apps von der kontinuierlichen Verbesserung gleichermaßen profitieren.

### Bezugszeichenliste

- 1: Farbindikator
- 2: Indikatorfeld
- 3: Grundträger
- 4: Farbreferenz
- 5: Frittierfett
- 6: Referenzfeld
- 7: erste Endfarbe
- 8: zweite Endfarbe
- 9: Farbabgleichsfeld
- 10: Markierung
- 11: Bilderfassungsschritt
- 12: Skalaschritt
- 13: Farbabgleichsschritt
- 14: Indikatorschritt
- 15: Modellschritt
- 16: Auswerteschritt
- 17: Messwert
- 18: Bild
- 19: Vorrichtung
- 20: Bilderfassungseinheit
- 21: Auswerteeinheit

## Patentansprüche

1. Verfahren zum Bestimmen des Messwertes einer Messgröße einer Substanz mit Hilfe eines Farbindikators (1), wobei der Farbindikator (1) wenigstens ein Indikatorfeld (2) aufweist, das zuvor mit der Substanz in Kontakt gebracht wurde, wobei der Farbindikator (1) und eine Farbreferenz (4) bildlich (18) erfasst (11) werden, wobei die Farbreferenz (4) wenigstens zwei verschiedenfarbige Referenzfelder (6) aufweist, **dadurch gekennzeichnet, dass** computerunterstützt anhand der aufgenommenen Farbwerte der Referenzfelder (6) und des Indikatorfeldes (2) ein Zwischenwert der Messgröße ermittelt wird (15), wobei jeweils zwei verschiedenfarbige Referenzfelder (6) die Endfarben (7, 8) einer Farbskala definieren und die Endfarben (7, 8) jeweils einem Endwert des Messbereichs des Farbindikators (1) zugeordnet sind und wobei der Zwischenwert durch Interpolation des Farbwertes des Indikatorfeldes (2) innerhalb der Farbskala und der zugeordneten Endwerte ermittelt wird, und dass der Messwert der Substanz aus diesem Zwischenwert ermittelt wird (16).

2. Verfahren nach Anspruch 1, **dadurch gekennzeichnet, dass** das Ermitteln (16) des Messwertes aus dem Zwischenwert anhand von statistischen Methoden und/oder durch eine künstliche Intelligenz erfolgt.

3. Verfahren nach Anspruch 2, **dadurch gekennzeichnet, dass** die künstliche Intelligenz (16) als Eingang den Farbwert und/oder den Zwischenwert jedes Indikatorfeldes (2) und den Farbwert jedes Referenzfeldes (6) erhält und als Ausgang den Messwert der Substanz liefert.

4. Verfahren nach einem der Ansprüche 1 bis 3, **dadurch gekennzeichnet, dass** ein automatischer Farbabgleich, insbesondere Weißabgleich, vorgenommen wird, insbesondere wobei die Farbreferenz wenigstens ein Farbabgleichsfeld (9) aufweist und/oder dass die Farbwerte der Farbabgleichsfelder (9) als weitere Eingänge der künstlichen Intelligenz (16) dienen.

5. Verfahren nach einem der Ansprüche 1 bis 4, **dadurch gekennzeichnet, dass** die bildliche Erfassung (11) des Farbindikators (1) und der Farbreferenz (4) gleichzeitig, insbesondere in einem Bild (18), erfolgt.

6. Verfahren nach einem der Ansprüche 1 bis 5, **dadurch gekennzeichnet, dass** der Farbindikator (1) mehr als ein Indikatorfeld (2), insbesondere vier Indikatorfelder (2), aufweist, insbesondere wobei die Indikatorfelder (2) unterschiedliche Sensitivitäten gegenüber der zu messenden Substanz aufweisen.

7. Verfahren nach einem der Ansprüche 1 bis 6, **dadurch gekennzeichnet, dass** die Farbreferenz (4) mehr als ein Referenzfeld (6) für jede Endfarbe (7, 8) aufweist, wobei die Referenzfelder (6) einer Endfarbe (7, 8) jeweils identische Farben aufweisen, insbesondere wobei für jede Endfarbe (7, 8) zwei Referenzfelder (6) vorhanden sind, die voneinander beabstandet und abwechselnd angeordnet sind.

8. Verfahren nach einem der Ansprüche 1 bis 7, **dadurch gekennzeichnet, dass** der Farbindikator (1) so ausgebildet ist, dass die zu messende Substanz so gebunden wird, dass keine Reflexionen durch Umgebungslicht entstehen und/oder dass Mittel zum Erkennen störender Reflexionen vorhanden sind, so dass eine Warnung ausgegeben werden kann.

9. Verfahren nach einem der Ansprüche 1 bis 8, **dadurch gekennzeichnet, dass** der Farbindikator (1) und die Farbreferenz (4) einteilig oder mehrteilig ausgebildet sind und/oder dass die Größe und die Abstände der Referenzfelder (6) der Größe und den Abständen der Indikatorfelder (2) angepasst sind, insbesondere wobei die Abstände identisch sind, so dass die Referenzfelder (6) jeweils neben ein Indikatorfeld (2) platzierbar sind.

10. Vorrichtung (19) zum Bestimmen des Messwertes einer Eigenschaft einer Substanz mit Hilfe eines Farbindikators (1) und einer Farbreferenz (4), wobei die Vorrichtung (19) eine Bilderfassungseinheit (20) zur bildlichen Erfassung des Farbindikators (1) und einer Farbreferenz (4), und eine Auswerteeinheit (21) zur computerunterstützten Ermittlung eines Zwischenwertes anhand der aufgenommenen Farbwerte der Referenzfelder (6) der Farbreferenz (4) und eines Indikatorfeldes (2) des Farbindikators (1) enthält, 11
dadurch charakterisiert dass
jeweils zwei verschiedenfarbige Referenzfelder (6) die Endfarben (7, 8) einer Farbskala definieren und die Endfarben (7, 8) jeweils einem Endwert des Messbereichs des Farbindikators (1) zugeordnet sind und wobei die Vorrichtung so ausgebildet ist, dass die Ermittlung des Zwischenwertes durch Interpolation des Farbwertes des Indikatorfeldes (2) innerhalb der Farbskala und der zugeordneten Endwerte erfolgt, und der Messwert der Substanz aus diesem Zwischenwert bestimmt wird .

11. Vorrichtung (19) nach Anspruch 10, **dadurch gekennzeichnet, dass** die Vorrichtung (19) ein Smartphone, ein Tablet oder ein Notebook ist, bei dem die Auswerteeinheit (21) durch ein Softwareprogramm oder eine App ausgebildet ist.

## Claims

1. Method for determining the measurement value of a measurement variable of a substance with the aid of a colour indicator (1), wherein the colour indicator (1) has at least one indicator field (2) which has previously been brought into contact with the substance, wherein the colour indicator (1) and a colour reference (4) are captured (11) in image-type fashion (18), wherein the colour reference (4) has at least two reference fields (6) of different colours, **characterized in that** an intermediate value of the measurement variable is determined (15) in a computer-aided manner on the basis of the recorded colour values of the reference fields (6) and the indicator field (2), wherein two reference fields (6) of different colours each define the end colours (7, 8) of a colour scale, and the end colours (7, 8) are each assigned to an end value of the measurement range of the colour indicator (1), and wherein the intermediate value is determined by interpolation of the colour value of the indicator field (2) within the colour scale and the assigned end values, and **in that** the measurement value of the substance is determined (16) from this intermediate value.

2. Method according to Claim 1, **characterized in that** the determining (16) of the measurement value from the intermediate value is effected on the basis of statistical methods and/or by means of artificial intelligence.

3. Method according to Claim 2, **characterized in that** the artificial intelligence (16) receives the colour value and/or the intermediate value of each indicator field (2) and the colour value of each reference field (6) as input and produces the measurement value of the substance as output.

4. Method according to any of Claims 1 to 3, **characterized in that** an automatic colour balance, in particular white balance, is performed, in particular wherein the colour reference has at least one colour balance field (9), and/or **in that** the colour values of the colour balance fields (9) serve as further inputs of the artificial intelligence (16).

5. Method according to any of Claims 1 to 4, **characterized in that** the image-type capture (11) of the colour indicator (1) and the colour reference (4) is effected simultaneously, in particular in one image (18).

6. Method according to any of Claims 1 to 5, **characterized in that** the colour indicator (1) has more than one indicator field (2), in particular four indicator fields (2), in particular wherein the indicator fields (2) have different sensitivities vis-à-vis the substance to be measured.

7. Method according to any of Claims 1 to 6, **characterized in that** the colour reference (4) has more than one reference field (6) for each end colour (7, 8), wherein the reference fields (6) of an end colour (7, 8) each have identical colours, in particular wherein two reference fields (6) arranged alternately and at a distance from one another are present for each end colour (7, 8).

8. Method according to any of Claims 1 to 7, **characterized in that** the colour indicator (1) is configured such that the substance to be measured is bound such that no reflections resulting from ambient light arise, and/or **in that** means for detecting disturbing reflections are present so that a warning can be emitted.

9. Method according to any of Claims 1 to 8, **characterized in that** the colour indicator (1) and the colour reference (4) are configured integrally or in multipartite fashion, and/or **in that** the size and the spacings of the reference fields (6) are adapted to the size and the spacings of the indicator fields (2), in particular wherein the spacings are identical, such that the reference fields (6) are each able to be placed next to an indicator field (2).

10. Device (19) for determining the measurement value of a property of a substance with the aid of a colour indicator (1) and a colour reference (4), wherein the device (19) contains an image capture unit (20) for the image-type capture of the colour indicator (1) and a colour reference (4), and an evaluation unit (21) for determining an intermediate value in a computer-aided manner on the basis of the recorded colour values of the reference fields (6) of the colour reference (4) and an indicator field (2) of the colour indicator (1), **characterized in that** two reference fields (6) of different colours each define the end colours (7, 8) of a colour scale, and the end colours (7, 8) are each assigned to an end value of the measurement range of the colour indicator (1), and wherein the device is configured such that the determination of the intermediate value is effected by interpolation of the colour value of the indicator field (2) within the colour scale and the assigned end values, and the measurement value of the substance is determined from this intermediate value.

11. Device (19) according to Claim 10, **characterized in that** the device (19) is a smartphone, a tablet or a notebook, in which the evaluation unit (21) is formed by a software program or an app.

## Revendications

1. Procédé pour définir la valeur de mesure d'une grandeur de mesure d'une substance à l'aide d'un indicateur chromatique (1), selon lequel l'indicateur chromatique (1) comporte au moins un champ indicateur (2) qui a été précédemment mis en contact avec la substance, selon lequel l'indicateur chromatique (1) et une référence chromatique (4) sont enregistrés (11) sous forme d'image (18) et selon lequel la référence chromatique (4) présente au moins deux champs de référence (6) de couleurs différentes, **caractérisé en ce qu'**une valeur intermédiaire de la grandeur de mesure est déterminée sur ordinateur à l'aide des valeurs chromatiques enregistrées des champs de référence (6) et du champ indicateur (2), deux champs de référence (6) de couleurs différentes définissant chacun les couleurs terminales (7, 8) d'une échelle chromatique, lesquelles couleurs terminales (7, 8) sont chacune associées à une valeur terminale de la plage de mesure de l'indicateur chromatique (1), et la valeur intermédiaire étant déterminée par extrapolation de la valeur chromatique du champ indicateur (2) à l'intérieur de l'échelle chromatique et des valeurs terminales associées, et que la valeur de mesure de la substance est déterminée (16) à partir de cette valeur intermédiaire.

2. Procédé selon la revendication 1, **caractérisé en ce que** la détermination (16) de la valeur de mesure à partir de la valeur intermédiaire est réalisée à l'aide de méthodes statistiques et/ou par une intelligence artificielle.

3. Procédé selon la revendication 3, **caractérisé en ce que** l'intelligence artificielle (16) reçoit en entrée la valeur chromatique et/ou la valeur intermédiaire de chaque champ indicateur (2), ainsi que la valeur chromatique de chaque champ de référence (6), et livre à la sortie la valeur de mesure de la substance.

4. Procédé selon une des revendications 1 à 3, **caractérisé en ce qu'**un équilibrage chromatique automatique, en particulier un équilibrage des blancs, doit être effectué, en particulier selon lequel la référence chromatique comporte au moins un champ d'équilibrage chromatique (9), et/ou que les valeurs chromatiques des champs d'équilibrage chromatique (9) servent d'entrées supplémentaires à l'intelligence artificielle (16).

5. Procédé selon une des revendications 1 à 4, **caractérisé en ce que** l'enregistrement visuel (11) de l'indicateur chromatique (1) et de la référence chromatique (4) a lieu en même temps, en particulier dans une image (18).

6. Procédé selon une des revendications 1 à 5, **caractérisé en ce que** l'indicateur chromatique (1) comporte plus d'un champ d'indicateur (2), en particulier quatre champs d'indicateur (2), en particulier ces champs d'indicateur (2) présentant des sensibilités différentes par rapport à la substance à mesurer.

7. Procédé selon une des revendications 1 à 6, **caractérisé en ce que** la référence chromatique (4) comporte plus d'un champ de référence (6) pour chaque couleur terminale (7, 8), selon lequel les champs de référence (6) d'une couleur terminale (7, 8) présentent chacun des couleurs identiques, en particulier selon lequel il existe pour chaque couleur terminale (7, 8) deux champs de référence (6) qui sont distants l'un de l'autre et disposés de façon alternée.

8. Procédé selon une des revendications 1 à 7, **caractérisé en ce que** l'indicateur chromatique (1) est configuré de telle sorte que la substance à mesurer est liée de telle sorte qu'aucun reflet n'est produit par la lumière environnante et/ou que des moyens pour identifier des reflets gênants sont disponibles, de sorte qu'un avertissement peut être émis.

9. Procédé selon une des revendications 1 à 8, **caractérisé en ce que** l'indicateur chromatique (1) et la référence chromatique (4) sont configurés en une ou plusieurs parties et/ou que la taille et les distances des champs de référence (6) sont adaptées à la taille et aux distances des champs indicateurs (2), en particulier selon lequel les distances sont identiques, de sorte que les champs de référence (6) peuvent être placés chacun près d'un champ indicateur (2).

10. Dispositif (19) pour définir la valeur de mesure d'une propriété d'une substance à l'aide d'un indicateur chromatique (1) et d'une référence chromatique (4), lequel dispositif (19) contient une unité d'imagerie (20) pour l'enregistrement visuel de l'indicateur chromatique (1) et d'une référence chromatique (4), et une unité d'évaluation (21) pour la détermination assistée par ordinateur d'une valeur intermédiaire à l'aide des valeurs chromatiques enregistrées des champs de référence (6) de la référence chromatique (4) et d'un champ indicateur (2) de l'indicateur chromatique (1), **caractérisé en ce que** deux champs de référence (6) de couleurs différentes définissent chacun les couleurs terminales (7, 8) d'une échelle chromatique et les couleurs terminales (7, 8) sont associées chacune à une valeur terminale de la plage de mesure de l'indicateur chromatique (1), lequel dispositif est configuré de telle sorte que la détermination de la valeur intermédiaire se fait par extrapolation de la valeur chromatique du champ indicateur (2) à l'intérieur de l'échelle chromatique et des valeurs terminales associées, et que la valeur de mesure de la substance est définie à partir de cette valeur intermédiaire.

11. Dispositif (19) selon la revendication 10, **caractérisé en ce que** ce dispositif (19) est un téléphone portable, une tablette ou un ordinateur portable dans lequel l'unité d'évaluation (21) est constituée par un programme informatique ou une application.
